# EUROPEAN PATENT APPLICATION

(11) **EP 0 810 004 A2**
(43) Date of publication of application: **03.12.1997**
(21) Application number: 97303128.9
(22) Date of filing: 08.05.1997
(51) Int. Cl.: A61M 29/00, A61N 5/10

(54) **Radiation-emitting flow-through temporary stent**

(30) Priority: 29.05.1996 US 654698
(71) Applicant: ADVANCED CARDIOVASCULAR SYSTEMS, INC., Santa Clara California 95052 (US)
(72) Inventor: Loeffler, Joseph P., Mountain View, California 94041 (US)
(74) Representative: McLeish, Nicholas Alistair Maxwell

(57) **Abstract**

The invention is directed to an intravascular catheter with an expandable region suitable for maintaining the patency of a body lumen for a period of time sufficient to permit delivery of a radiation source to the body lumen. The expandable region centers the radiation dose within the body lumen, such as a coronary artery, and permits blood flow through the expandable region while radiation therapy is provided.

## Description

### BACKGROUND OF THE INVENTION

This invention generally relates to intravascular catheters suitable for maintaining the patency of a body lumen for a period of time sufficient to permit delivery of a radiation source to the body lumen.

In typical percutaneous transluminal coronary angioplasty (PTCA) procedures, a guiding catheter having a preformed distal tip is introduced percutaneously into the cardiovascular system of a patient through the brachial or femoral arteries and is advanced until the distal tip of the guiding catheter is in the ostium of the desired coronary artery. A guide wire, and a dilatation catheter having an inflatable balloon on the distal end thereof, are introduced through the guiding catheter with the guide wire slidably disposed within an inner lumen of the dilatation catheter. The guide wire first is advanced out of the distal end of the guiding catheter and then is maneuvered into the coronary vasculature of a patient where the lesion to be dilated is located, and then is advanced beyond the lesion. Thereafter, the dilatation catheter is advanced over the guide wire until the dilatation balloon is positioned across the lesion. Once in position across the lesion, the balloon of the dilatation catheter is filled with radiopaque liquid at relatively high pressures (e.g., greater than about 4.05 bars (4 atmospheres)) and is inflated to a pre-determined size (preferably the same size as the inner diameter of the artery at that location), in order to radially compress the atherosclerotic plaque of the lesion against the inside of the artery wall to thereby dilate the lumen of the artery. The balloon then is deflated so that the dilatation catheter can be removed and blood flow can resume through the dilated artery.

A common problem that sometimes occurs after an angioplasty procedure is the appearance of restenosis at or near the site of the original stenosis in the body lumen. Such restenosis usually requires a secondary angioplasty procedure or bypass surgery.

In recent years, various devices and methods (other than bypass surgery) for the prevention of restenosis after arterial interventions in the body lumen of a patient have become known. Typically, such devices and methods involve use of an expandable cage or an apparatus commonly referred to as a "stent" on the distal end of the catheter. Stents normally are designed for long-term implantation with the body lumen; and some stents are intended for permanent implantation within the body lumen. By way of example, several stent devices and methods can be found in commonly assigned and commonly owned U.S. Patent No. 5,002,560 (Machold et al.), U.S. Patent No. 5,034,001 (Garrison et al.), U.S. Patent No. 5,180,368 (Garrison), U.S. Patent No. 5,263,963 (Garrison et al.), and U.S. Patent No. 5,456,667 (Ham et al.).

More recently, devices and methods to counteract the biological process of restenosis after arterial intervention have employed a radiation source delivered through a balloon catheter to the area of the body lumen affected by the restenosis. The radiation is intended to target and destroy the cell growth responsible for the restenosis. Two such devices and methods are described in International Publication No. WO 93/04735 (Hess) and WO 95/19807 (Weinberger).

What has been needed and what has been heretofore unavailable is a catheter with an expandable region that can hold open the area of an artery where restenosis is likely to occur to allow delivery of a radiation source to the area of the restenosis for a period of time sufficient to destroy the cells of the restenosis while still allowing perfusion of blood in the affected area during irradiation. Such an intravascular catheter must be easy and inexpensive to manufacture, and must have an expandable region that is strong and reliable under pressure, and must be capable of being formed in a variety of shapes to allow flexibility in the amount and pattern of expansion and deformation of the expandable region. Further, the associated radiation source with which the catheter is used must be protected from any contact with the body fluids of the patient, so as to allow it to be used again with other patients.

### SUMMARY OF THE INVENTION

Particular embodiments of the invention are directed to an intravascular catheter with an expandable region located at the distal end of the catheter body which expandable region can hold a body lumen open for a period of time that is sufficient to permit delivery of a radiation source to a body lumen while permitting perfusion of blood through the vessel.

An intravascular catheter embodying the invention includes an elongated catheter body formed with a member having a control wire lumen which extends through the entire length of the body; a guide wire lumen in the distal portion of the catheter body, adapted to receive a guide wire therein, and which extends through the distal portion from a proximal opening in the sidewall of the distal portion to an opening in the distal end of the elongated catheter body; and a "blind" lumen, adapted to receive a radiation source in the form of a wire, which extends from the proximal end of the elongated catheter body to an area near the distal end of the elongated catheter body, which distal end is sealed to prevent communication of any body fluids of the patient with the blind lumen.

An expandable region is attached to the distal end of the elongated catheter body. The proximal end of the expandable region begins at the distal end of the elongated catheter body and the distal end of the expandable region is not attached to the elongated catheter body. Accordingly, the proximal end of the expandable region is fixed in place, but the distal end of the expandable region is free to move longitudinally, relative to the elongated catheter body.

A control wire extends through the control wire lumen of the elongated catheter body and into the interior of the expandable region, with the distal end of the wire connected to the distal collar which is secured to the distal end of the expandable region. A flexible tubular guide, such as a coiled spring or a flexible tubular member, is provided on the interior of the expandable region between the ends thereof to ensure the proper passage of the guide wire therethrough. If not properly guided, the guide wire can diverge out of its travel path and move toward the inside of the expandable region. Longitudinal movement of the control wire forces movement of the distal collar and the distal end of the expandable region that is connected to the distal collar. Such movement adjusts the axial spacing between the proximal and distal ends of the expandable regions. When the control wire is moved proximally, the tubular material forming the expandable region deforms to a larger diameter. When the control wire is extended distally, the tubular material forming the expandable region will extend to its original diameter. Preferably, the control wire is sufficiently stiff so that movement thereof in the distal direction will cause the expandable region to elongate without bending or kinking of the wire. This eliminates the need for biasing the expandable region in some manner so as to return it to an elongated state with minimal radial dimensions after the expansion thereof to permit the catheter to be removed from the blood vessel. A suitable manipulator is provided on the proximal end of the catheter assembly to longitudinally move the control wire within the first lumen of the tubular member.

The expandable region is configured to be flexible so that it can be expanded on a curved portion of a body lumen, such as a coronary artery. It is also configured to center the radiation source wire within the body lumen, even if the expandable region is positioned on a curved section of the body lumen.

The relatively short guide wire lumen disposed within the distal portion of the elongated catheter body preferably is defined in part by a sidewall in the distal portion of the tubular member which is provided within an elongated slot extending distally from the proximal hole in the sidewall to a location proximally adjacent the proximal end of the expandable region. This slotted construction greatly facilitates the rapid exchange of the vascular device of the invention over an in-place guide wire, in the event it is desired to perform redundant or additional procedures at the same site in the body lumen.

The proximal opening or port of the guide wire lumen should be spaced proximally more than about 15 cm but less than about 60 cm, preferably from about 20 cm to about 50 cm, from the distal end of the elongated catheter body, to ensure that the proximal opening in the sidewall of the elongated catheter body does not extend beyond the distal end of the guide wire during a vascular procedure. If the guide wire is not restrained in some manner, it will tend to loop back on itself as the intravascular catheter of the invention is pulled proximally to withdraw it from the patient. Loop formation can interfere with the subsequent removal of the elongated catheter body through the guiding catheter.

In the presently preferred embodiment aforedescribed, a third lumen, which has a proximal end and a distal end, is provided in the elongated catheter body which extends from the proximal end of the elongated catheter body to a location approximately centered within the expandable region at the distal end of the elongated catheter body. This lumen is a "blind" lumen (or "dead-end" lumen) in that it is closed and sealed at the distal end to prevent patient body fluids, such as blood, from entering into it. This blind lumen allows advancement of a radiation source wire from the proximal end of the elongated catheter body to a location near the distal end of the blind lumen and within the expandable region of the catheter. When the expandable region is expanded into contact with the body lumen, the radiation source wire will be centered in the body lumen and a radiation dose can be administered over a long period of time. The expanded region permits perfusion of blood flow during the procedure thereby allowing longer time periods of radiation exposure. With the described embodiment, lower levels of radiation can be used for longer time periods to provide the necessary dosage.

In one embodiment of the invention, the expandable region is formed from or is coated or impregnated with the radiation source, thereby eliminating the need for a blind lumen and radiation source wire. When the expandable region is expanded into contact with the body lumen, the radiation source also comes in contact with the body lumen. Centering a source wire is unnecessary with this embodiment, while perfusion of blood is still maintained. In this embodiment the radiation source is exposed to the patient's blood and is thus not reusable.

An intravascular catheter embodying the invention also allows for an over-the-wire delivery of the elongated catheter body to a location within a body lumen wherein the radiation dose is to be administered. A guide wire lumen extends from the proximal end of the catheter body all the way through and out its distal end and is dimensioned to slide over the in-place guide wire. The expandable region, when expanded, will hold the body lumen open and simultaneously allow blood flow through the expandable region thereby eliminating or preventing ischemic conditions while providing sufficient time for the radiation source to provide the required dose to abate the restenosis related cell growth in the area of the body lumen affected.

According to another aspect of the present invention there is provided a method for maintaining the patency of a body lumen for a period of time sufficient to permit delivery of a radiation source to the body lumen, comprising: providing a catheter having: an elongated catheter body having a proximal end and a distal end; an expandable region disposed at the distal end of the elongated catheter body, the expandable region having a proximal end and a distal end; a guide wire lumen extending through the elongated catheter body from the proximal end to the distal end for receiving a guide wire; means for expanding and contracting the expandable region; and a blind lumen disposed in the elongated catheter body and extending from the proximal end and terminating at a position near the distal end of the expandable region, the blind lumen adapted to receive a radiation source wire; positioning the guide wire in the body lumen; advancing the catheter over the guide wire by inserting the guide wire in the distal end of the catheter and into the guide wire lumen; advancing the elongated catheter body over the guide wire until the expandable region is positioned in the body lumen; expanding the expandable region into contact with the body lumen; centering the blind lumen in the body lumen; perfusing blood flow through the expandable region; inserting the radiation source wire in the blind lumen for delivering a radiation dose to the body lumen; contracting the expandable region; and withdrawing the catheter and the radiation source wire from the body lumen.

Advantageously the means for expanding and contracting the expandable region includes providing a control wire lumen disposed in the elongated catheter and extending from the proximal end to the expandable region, the control wire lumen being adapted to receive a control wire for effecting axial movement of the expandable region. Preferably the control wire is attached to the distal end of the expandable region, the method further comprising withdrawing the control wire proximally to shorten the expandable region thereby expanding it into contact with the body lumen, the distal end of the expandable region moving axially in the proximal direction while the proximal end of the expandable region remains fixed relative to the elongated catheter body.

Advantageously the means for expanding and contracting the expandable region includes providing the elongated catheter body with an outer shaft and an inner shaft the method further comprising expanding and contracting the expandable region by providing relative axial movement between the outer shaft and the inner shaft. Preferably the relative axial movement between the outer shaft and the inner shaft is controlled by manipulating a rack and pinion. Alternatively the relative axial movement between the outer shaft and the inner shaft is controlled by manipulating ratcheting means. Alternatively the relative axial movement between the outer shaft and the inner shaft is controlled by manipulating gearing means.

Advantageously the radiation source wire delivers a low dose of radiation.

Advantageously the radiation dose is delivered intraluminally into the coronary arteries.

Advantageously the radiation dose delivered is in the range of 20 to 3000 rads.

Advantageously the radiation dose is exposed to the body lumen for no less than one minute.

Advantageously the radiation dose is taken from the group of iridium¹⁹², cobalt⁶⁰, vanadium⁴⁸, gold¹⁹⁸, and phosphorus³².

Advantageously the step of providing the catheter further includes a plurality of expandable members for centering the radiation source wire in the body lumen.

These and other advantages of the invention will become more apparent from the following detailed description thereof when taken in conjunction with the accompanying exemplary drawings.

### BRIEF DESCRIPTION OF THE DRAWINGS

FIGURE 1 is an elevational view, partially in cross-section, of an intravascular catheter of rapid exchange design embodying features of the invention.

FIG. 1a is an elevational view of the catheter shown in FIG. 1 depicting the expandable region in its expanded condition.

FIG. 2 is a cross-sectional view of the catheter of FIG. 1 taken along lines 2-2.

FIG. 3 is a cross-sectional view of the catheter expandable region of FIG. 2 taken along lines 3-3.

FIG. 3a is a cross-sectional view of FIG. 1a taken along lines 3a-3a, depicting the expandable region in its expanded condition.

FIG. 4 is an elevational view of one embodiment of the expandable region in its unexpanded condition.

FIG. 4a is an elevational view of the expandable region of FIG. 4, in its expanded condition.

FIG. 5 is an elevational view of one embodiment of an intravascular catheter of over-the-wire design with a wire cage expandable region.

FIG. 6. is a cross-sectional view of the catheter of FIG. 5, taken along lines 6-6, depicting the control wire lumen, the guide wire lumen, and the radiation source wire lumen.

FIG. 6a is a cross-sectional view of the wire cage expandable region of the catheter of FIG. 5, taken along lines 6a-6a, depicting the various catheter lumens and the expanded region being fully expanded.

FIG. 6b is a cross-sectional view of the wire cage expandable region of the catheter having multiple expandable regions which are fully expanded.

FIG. 6c is a cross-sectional view of the wire cage expandable region of the catheter of FIG. 6b, in which the multiple expandable regions are expanded within a curved section of artery thereby centering the radiation source wire.

FIG. 7 is an elevational view of another embodiment of an over-the-wire catheter wherein an inner and an outer member control the expandable region of the catheter.

FIG. 7a is a cross-sectional view of the catheter of FIG. 7, taken along lines 7a-7a, depicting the coaxial arrangement of the inner and outer members and the source wire lumen.

### DETAILED DESCRIPTION OF THE PREFERRED EMBODIMENTS

Particular embodiments of the invention provide a catheter which is adapted to deliver a low dose radiation source to a body lumen, such as a coronary artery, for an extended period of time. The catheter permits perfusion of blood during the radiation therapy and will center the radiation source so that equal amounts of radiation are applied to the artery. While the embodiments of the invention are described in detail as applied to the coronary arteries, those skilled in the art will appreciate that it can be used in other body lumens as well, such as peripheral arteries and veins. Where different embodiments have like elements, like reference numbers have been used.

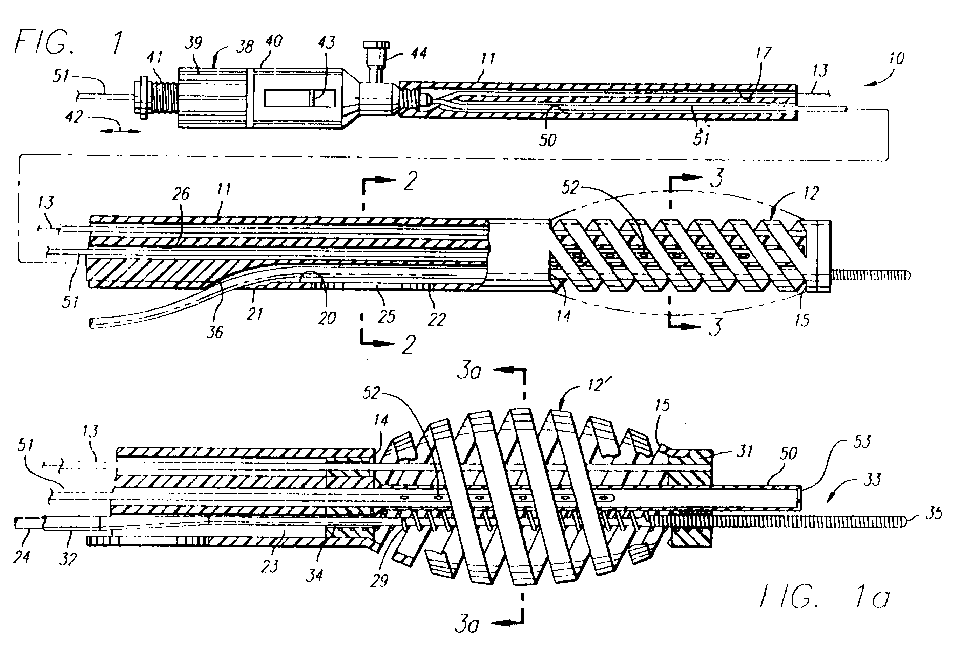

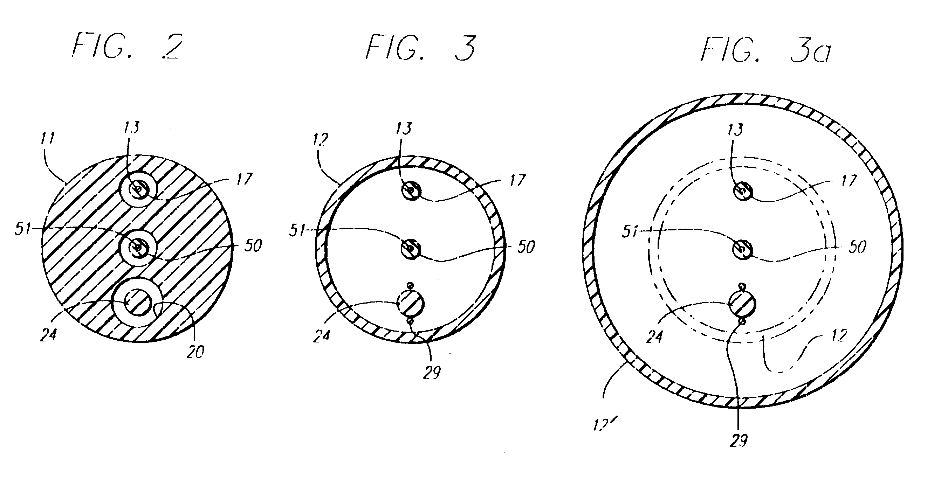

FIGS. 1-3a illustrate an intravascular catheter assembly 10 embodying features of the invention. The catheter assembly 10 generally includes an elongated catheter body 11, an expandable region 12 at the distal end of the catheter body, and a control wire or cable 13 for adjustment of the axial distance between a proximal end 14 and a distal end 15 of the expandable region 12 to vary the radial expansion thereof.

The elongated catheter body 11 has a control wire lumen 17 which extends through essentially the entire length thereof and which is adapted to receive the control wire 13. The catheter body 11 also includes a guide wire lumen 20 positioned in the distal portion of the catheter body which extends from a side port 21 in a sidewall 22 of the catheter body 11 to a port 23 provided in the distal end of the catheter body. A longitudinal slit 25 preferably is provided in the sidewall 22 which extends distally from the side port 21. A guide wire 24 is slidably disposed within the relatively short guide wire lumen 20 to facilitate the rapid advancement and replacement of the catheter assembly 10.

Further details of rapid exchange catheters can be found in U.S. Patent Nos. 5,458,613; 5,180,368; and 5,496,346. A blind lumen 50, which is provided within the catheter body 11, extends from the proximal end of the catheter body to a location proximate to the distal end of the expandable region. The blind lumen 50 is closed off at a distal end 53 thereof to seal it from communication with any body fluids such as blood. A radiation source wire 51, is inserted into the blind lumen for a period of time sufficient to deliver the required radiation dose to the body lumen. Preferably, the radiation source wire 51 is hollow at its distal end and contains a radiation dose in the form of radiation pellets 52, an irradiating gas, or a radioactive liquid or paste. The radiation source wire 51 also may have a radioactive source coated on its distal end.

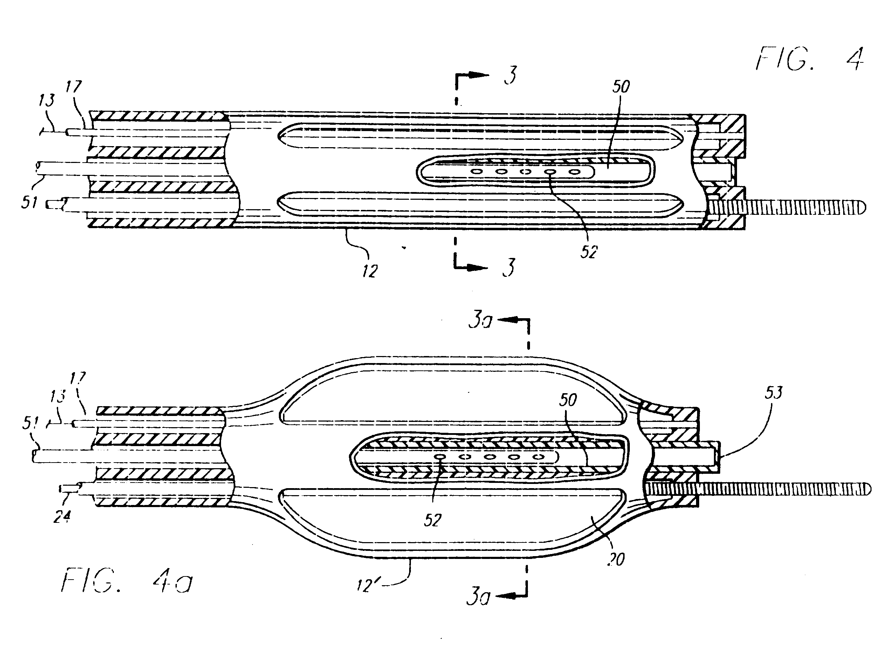

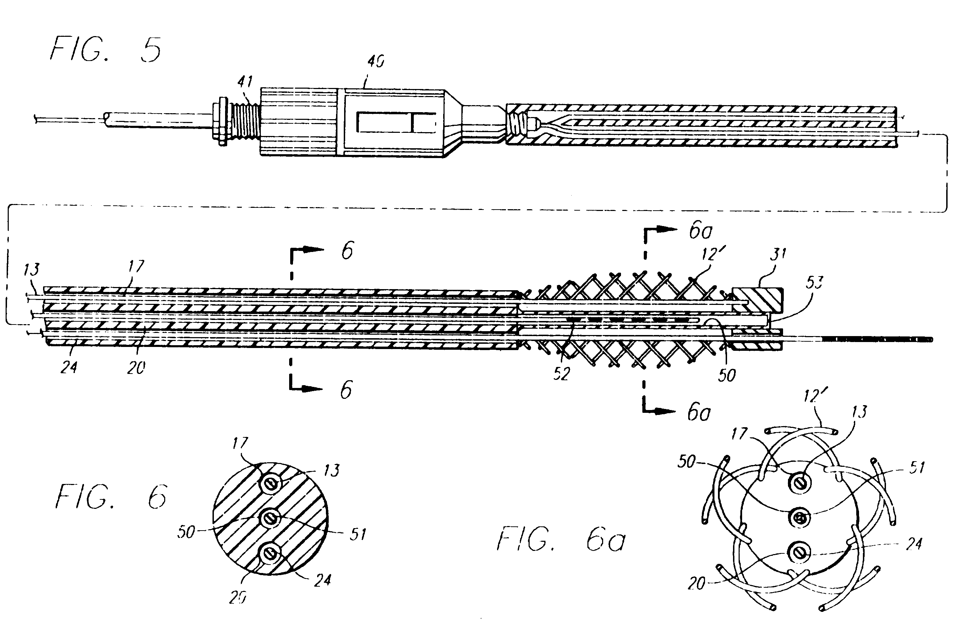

The patterns of the expandable region 12 can vary considerably as long as perfusion of blood through the expanded region is maintained. In a presently preferred embodiment, shown in FIG. 1, a spiral pattern is created in the tubular body forming the expandable region 12. By way of example, two other embodiments of many possible patterns for the formation of the expandable region are shown in FIGS. 4, 4a and 5. A flexible tubular member 29 is provided within the interior of the expandable region 12 between the proximal end 14 and the distal end 15 to align the guide wire 24 through the interior of the expandable region. The distal end 15 of the expandable region 12 is bonded by suitable means such as an adhesive to the distal collar 31 which has a passageway for the guide wire to be advanced therethrough. The distal end of the control wire 13 also is fixed to the distal collar 31, which is slidably contained within the first inner lumen 17 so that longitudinal or axial movement of the control wire adjusts the axial spacing between the proximal end 14 and the distal end 15 of the expandable region, thereby varying the radial dimension of the expandable region.

The guide wire 24 comprises a core member 32, a helical coil 33 or other flexible body disposed about and fixed to a tapered distal portion 34 of the core member. A rounded plug 35, preferably formed of a radiopaque material, is provided at the distal tip of the coil 33. The construction of the distal portion of the guide wire 24 can have a conventional structure with the core member 32 extending through the helical coil 33 to the plug 35, or the distal portion can be constructed so that the core member terminates short of the plug 35 and a shaping ribbon (not shown) extends from the core member 32 to the plug 35. The guide wire 24 extends through the guide wire lumen 20 disposed within the distal portion of the elongated catheter body 11 and out the distal port 23, through the flexible tubular guiding element 29 which extends through the interior of the expandable region 12 and out of the distal end thereof, through the distal collar 31. An incline or ramp 36 is provided at the proximal end of the guide wire lumen 20 at the entryway of the side port 21 to facilitate the insertion and withdrawal of the guide wire 24 therethrough.

The distance between the distal end 15 of the expandable region 12 and the side port 21 should be at least 15 cm but not greater than 60 cm, preferably from about 20 to about 50 cm, so that when the expandable region is expanded within a patient's vascular system to hold a blood vessel open, the side port 21 of the elongated catheter body 11 will remain within the interior of a guiding catheter to ensure that the guide wire 24 does not have the opportunity to form a loop when the catheter assembly is pulled back into the guiding catheter.

A manipulator adapter 38 is provided on the proximal end of the catheter body 11 to effect longitudinal movement of the control wire 13. An internally threaded cap 39 is secured to the proximal end of the manipulator housing 40. Axial rotation of the cap 39 causes longitudinal movement of the internal member 41 as shown by the arrow 42 in FIG. 1, and as a result, controls the axial spacing between the proximal end 14 and the distal end of the expandable region 12 and thus controls the radial dimension thereof. If the control wire 13 is relatively stiff, it can be employed to extend ends 14 and 15 of the expandable region 12 away from each other, elongating the expandable region so that it can be removed from the site of a blockage. If the control wire is not sufficiently stiff, the ease with which the expandable region is returned to its elongated state in preparation for removing the catheter from the patient's body can be enhanced by appropriately biasing the expandable region, so that upon release of the manipulator, the expandable region returns to its elongated condition. An indicator 43 is provided on the internal member 41 to display the radial dimension of the expandable region 12. Further details of the manipulator 38 can be round in U.S. Patent No. 5,002,560 entitled EXPANDABLE CAGE CATHETER WITH A ROTATABLE GUIDE.

Generally, the dimensions of the catheter assembly essentially are the same as the dimensions of vascular catheters commonly used in angioplasty procedures. The overall length of the assembly may be about 100 to 175 cm. The diameter of the catheter body 11 may range from about 0.254 mm to 1.524 mm (0.010 to 0.06 inch). The expandable region 12 in the unexpanded condition has approximately the same diameter as the catheter body but may be expanded to a maximum diameter of about 1 to about 10 mm. The diameter of control wire lumen 17 will depend upon the size of the control wire 13. The diameter of the guide wire lumen 20 should be sufficiently larger than the diameter of the guide wire 24 to allow the catheter to be easily advanced and removed over the guide wire.

In the preferred method of delivering a radioactive dose to a coronary artery, the guide wire 24 is positioned across the portion of the arterial passageway where a previous PCTA or atherectomy procedure has been performed. The proximal end of the guide wire is advanced proximally through the central passageway provided in the distal collar 31, guided through the interior of the expandable region 12 by the flexible tubular guiding element 29, through the port 23 leading into the guide wire lumen 20, through the guide wire lumen, and then out the side port 21. The proximal portion of the guide wire 24 extending out of the side port 21 then is manually held while the catheter assembly 10 is advanced over the guide wire through a previously positioned guiding catheter to a desired location within the blood vessel of the patient, such as where a prior vascular procedure has been performed. The cap 39 on the manipulator 38 is rotated to expand expandable region 12' (prime numbers indicate the expandable region in the expanded state) and thereby hold open the artery while maintaining the patency of the artery and allowing blood to flow through the expanded region. Once the expandable region 12' is expanded, the radiation source wire 51 is inserted into the proximal end of the blind lumen 50 and advanced until the radiation source 52, positioned at the distal end of radiation source wire 51, is positioned at that portion of the coronary artery which is intended to receive the radiation dose. The expandable region 12' is held in the expanded condition for a sufficient time, typically for one to five hours, to allow a sufficient radiation dose to kill the cells of the restenosis. Preferably, a sufficient dosage of radiation can be delivered from about one minute up to about sixty minutes. Treatments of greater periods are allowable because the expandable region design maintains patency of the artery during the treatment and allows blood flow on both sides and through the expandable region 12'. Further, in its expanded condition, the expandable region 12' presses against the walls of the artery and, in so doing, automatically enters the radiation source wire 51 relative to the walls of the artery. Centering the radiation dose is important so that all portions of the artery receive uniform and equal amounts of radiation therapy. During the period of expansion of the expandable region, blood flows readily through the openings in the expandable region so that no ischemia occurs distal to the catheter either in the artery or in any branches thereof.

After the radiation dose has been administered to the restenosis area, the radiation source wire 51 can be removed, the expanded region 12 can be elongated and contracted by rotating the cap 39 in a direction opposite to the direction for expanding the expandable region. Then the catheter assembly 10 can be withdrawn from the location within the vasculature of the patient.

Because of the design of the rapid exchange catheter assembly of FIGS. 1-3a, as the distal section of the catheter body emerges from the proximal end of the guiding catheter, the guide wire 24 can be separated from the guide wire lumen by pulling the guide wire through the slit 25 which extends from the side port 21 to a location adjacent the proximal end 14 of the expandable region 12. This allows the guide wire to be manually held exterior to the guiding catheter while the catheter assembly 10 is being exchanged for another catheter device if such an exchange proves to be necessary.

FIGS. 4 and 4a illustrate an alternative pattern for the expandable region 12. FIG. 4a depicts the expandable region 12' in the expanded position. This embodiment operates substantially the same as the embodiment of FIG. 1, and when expanded in a body lumen, will center the radiation source wire 51 in the artery and will permit blood flow through the expanded region while the radiation therapy is provided.

In another preferred embodiment of the invention, as depicted in FIGS. 5-6c, a wire mesh expandable region 12', is expanded to hold open an artery while the radiation dose wire 51 is inserted then advanced through the blind lumen 50. Radiation pellets 52 can be positioned at the appropriate location in the distal end of the radiation source wire 51 to deliver the radiation dose. As with all of the preferred embodiments, the expandable cage 12' permits blood to perfuse and to flow through the expandable cage 12' while it is expanded so that radiation source wire 51 can be inserted and left in place in the artery for a longer period of time without adverse affects to the patient. The expandable region 12' also centers the radiation source wire 51, and more specifically, the radiation pellets 52, so that uniform and equal amounts of radiation therapy are applied to the artery wall. More specifically, it would be undesirable to have the radiation source wire 51 not centered in the artery, because an uncentered radiation source may lead to radiation hot spots occuring on the arterial wall.

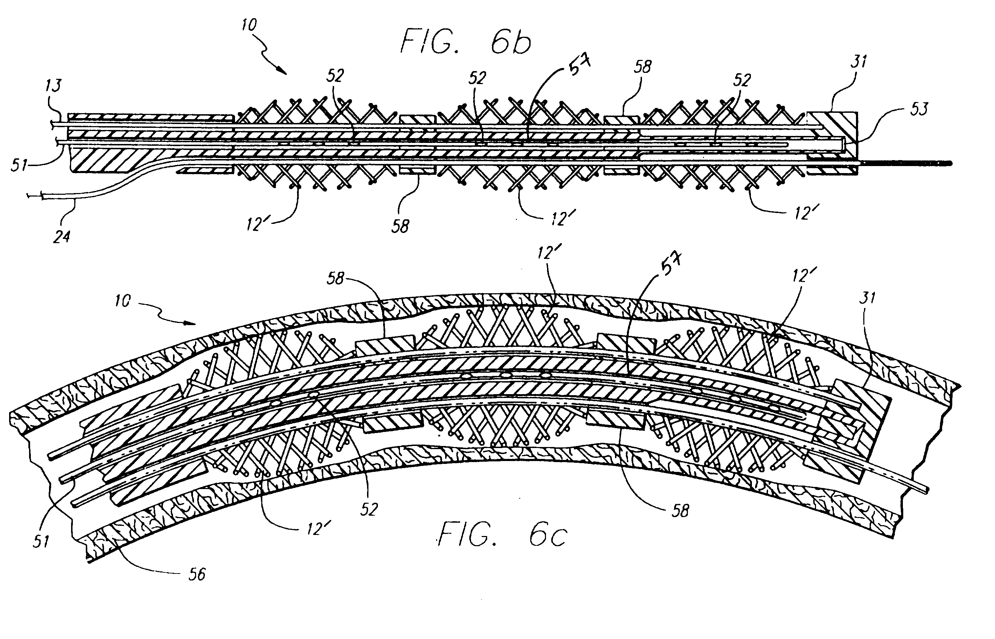

As can be seen in FIGS. 6b and 6c, multiple expandable regions or cages 12' are positioned along the distal portion of the catheter assembly 10. It is intended that this distal portion of the catheter assembly 10 be flexible so that it can easily navigate a tortuous artery as the catheter assembly is advanced along the guide wire 24. Further, it is important that the radiation source wire 51 be centered even when the area to which radiation is to be delivered is on a curved portion of an artery or vein. Accordingly, as depicted in FIG. 6c, the expandable regions or cages 12' are spaced apart such that radiation source wire 51 continues to be centered on the curved portion of the artery 56. Because the catheter assembly 10 in FIGS. 6b and 6c is flexible, it easily conforms to the curved portion of the artery 56, and the expandable region 12' expands into contact with the artery, thereby centering the radiation source wire 51 and hence the radiation pellets 52. In this manner, the radiation pellets 52 uniformly will deliver a radiation dose, in equal amounts, to all portions of the affected artery 56. Each of the embodiments as described herein can have the same configuration as that depicted in FIGS. 6b and 6c for the purpose of delivering a radiation dose on a curved portion of the artery 56.

In order to effectively expand the expandable region 12' of the embodiment of FIGS. 6b and 6c, it may be necessary to have multiple support collars 58 carried by an inner tubular member 57. The inner tubular member 57 has a blind lumen 50 formed therethrough, and is sealed at the distal end 53 of the blind lumen 50. Multiple support collars 58 are carried by inner tubular member, and provide the basis for guiding and carrying the control wire 13 and the guide wire 24. For example, the guide wire 13 is attached to each of the support collars 58 and the distal collar 31 so that when the control wire 13 is withdrawn proximally, it will expand the expandable cage or the expandable region 12' into contact with the artery 56. On the other hand, the guide wire 24 freely moves through the support collars 58 and the distal collar 31 so that the distal portion of the catheter assembly 10 easily can move to be advanced or withdrawn over the guide wire 24.

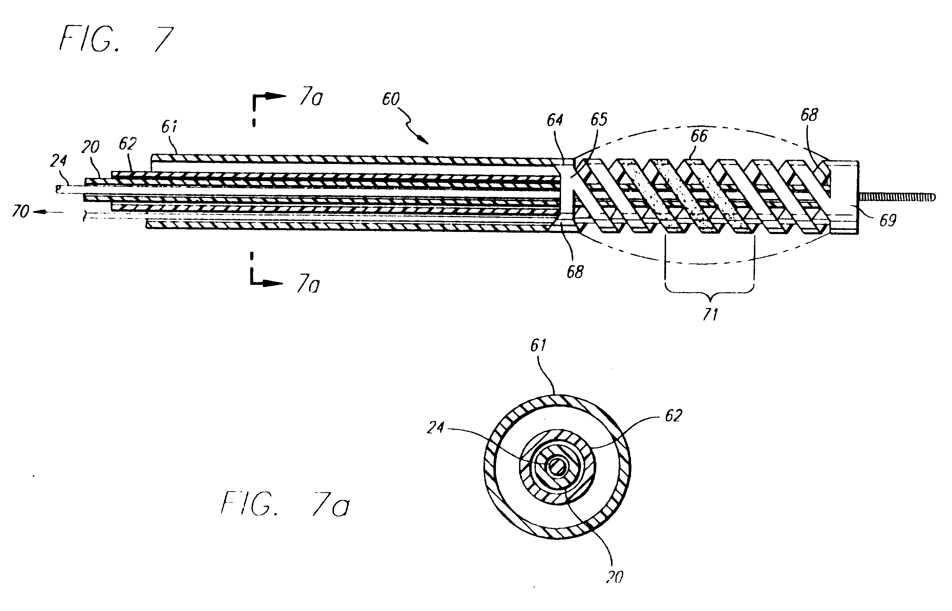

In another preferred embodiment of the invention, the expandable region of the catheter assembly is impregnated or coated with the radiation source. When the expandable region is expanded into contact with a body lumen, the radiation source is in contact with the wall of the body lumen and will kill those cells forming the restenosis. With this embodiment, a separate radiation source wire, as described above, is unnecessary. Further, it is unnecessary to center the radiation source, because it will be in contact with the body lumen. FIG. 7 illustrates this embodiment of the invention wherein the elongated catheter body 60 has an outer shaft 61 and an inner shaft 62 which run the entire length of the catheter body in a coaxial configuration. The outer shaft 61 has a proximal end (not shown) and a distal end 64 with the distal end 64 attached to a proximal end 65 of the expandable region 66. The inner shaft 62 has a proximal end (not shown) and a distal end 68 with the distal end 68 attached to a distal end 69 of the expandable region 66. The proximal ends of both the outer and inner shafts are attached to a structural means for providing relative axial movement between the outer shaft and the inner shaft for expanding and contracting the expandable region 66. A radiation source 71 either is impregnated in or coated on the expandable region 66. When the expandable region 66 is expanded, the radiation source 71 comes into contact, or near contact, with the arterial wall. Thus, the radiation source will kill those cells that are proliferating and causing the restenosis. As has been described, the expandable region is configured so that blood is permitted to flow through the expanded region during the radiation therapy treatment. This permits the physician to use a lower dosage of radiation for a longer period of time, so that no harmful effects of higher radiation doses are delivered to the patient.

In one preferred embodiment, the means for relative axial movement for the expandable regions includes a rack and pinion mechanism (not shown), attached to the proximal end 70 of the elongated catheter body 60. As a person skilled in the art would readily understand, to initiate relative axial motion between the outer shaft 61 and the inner shaft 62, a pinion gear is turned by a handle which engages upper and lower rack assemblies attached to the proximal ends of the outer and inner shafts respectively. A clockwise rotation of the pinion gear handle moves the engaged upper rack, and therefore the proximal end of the expandable region, towards the distal end 69 of the expandable region. At the same time, the lower rack, and therefore the distal end 69 of the expandable region 66, moves towards the proximal end 65 of the expandable region. This relative movement of the proximal end 65 and the distal end 69 of the expandable region towards each other shortens the region and causes it to expand. Conversely, a counter-clockwise rotation of the pinion gear handle moves the proximal and distal ends of the expandable region away from each other, thereby elongating the region and thus reducing its diameter.

A person skilled in the art of mechanics will readily identify that the rack and pinion mechanism herein described can be replaced by a screw-gear mechanism or a ratcheting mechanism to expand and contract the expandable region. Further details of the various configurations to impart relative axial movement between a coaxial inner and outer shaft can be found in the prior art and is well known.

A catheter assembly embodying the invention as described herein generally are employed after an atherectomy or percutaneous transluminal coronary angioplasty procedure (PTCA) to hold open an artery sufficiently long enough to allow a radiation dose to be administered to an area where restenosis invades the coronary artery. Furthermore, the expandable region of the catheter of the invention permits perfusion of blood through the expandable region during the entire radiation therapy process. It will be recognized by those skilled in the art that the catheter can be used within the vasculature system of a patient after vascular procedures other than a PTCA or an atherectomy.

A catheter assembly embodying invention may be formed of conventional materials of construction which are described in detail in the prior art patents referenced herein. The material forming the catheter body and the expandable region can be made of any metal or polymer with ductile properties which would be acceptable for the specific needs of intravascular devices. Specifically, the material chosen for the catheter body and the expandable region preferably would provide sufficient hoop strength for the expandable region to serve as a temporary stent while having enough flexibility to easily advance and navigate through tortuous anatomy. In addition, the portion of the material used to form the expandable region preferably would be sufficiently thin to allow the expandable region to expand easily and to permit blood flow therethrough. For example, the catheter body 11 and the expandable region 12 can be made of thin stainless steel tubing, nickel titanium alloy, polymer tubing or the like. A presently preferred material for the catheter body and the expandable region is stainless steel. The control wire 13 may be formed of stainless steel, but it may be formed of other materials such as titanium, nickel-titanium, and platinum-nickel alloys (e.g., 90 wt % Pt, 10 wt % Ni), or suitable polymers or even composites. Variations can be made in the composition of the materials to vary properties.

As described herein, a catheter assembly embodying the invention will deliver a low dosage of radiation to the body lumen, such as a coronary artery, and is configured to provide the dosage over longer periods of time than that disclosed by the prior art. It is preferred that a low dosage of radiation, on the order of .1 up to 3.0 curies be the typical radiation dosage provided to treat, for example, restenosis in a coronary artery. Preferably, 1.0 to 2.0 curies will provide the proper dose level.

The radiation delivered to a coronary artery should be in the range from about 20 to 3,000 rads in preferably not less than two minutes. The radiation dose can be delivered in less than two minutes, however, it is preferred that a longer time frame be used so that a lower dose can be administered.

It is contemplated that different radiation sources can be used, and the preferred radiation sources include iridium¹⁹², cobalt⁶⁰, vanadium⁴⁸, gold¹⁹⁸, and phosphorus³². It is also contemplated that whichever radiation source is used, that it have a half life of approximately less than one hundred days. Further, it is contemplated that the radiation sources emit either alpha or gamma particles to kill the target cells, however, beta-emitting radiation also can be used even though the radiation does not travel very far in human tissue. The use of alpha- and gamma-emitting radiation is well known for treating and killing cancerous cells.

Other modifications can be made to the present invention without departing from the scope thereof. The specific dimensions, dosages, times, and materials of construction are provided as examples and substitutes are readily contemplated which do not depart from the invention.

## Claims

1. An intravascular catheter (10) for maintaining the patency of a body lumen for a period of time sufficient to permit delivery of a radiation dose to the body lumen, comprising:
an elongated catheter body (11) having a proximal end and a distal end;
an expandable region (12) disposed at the distal end of the elongated catheter body, the expandable region having a proximal end (14) and a distal end (15);
a guide wire lumen (20) extending through the elongated catheter body (11) from the proximal end to the distal end for receiving a guide wire (24);
a control wire lumen (17) disposed in the elongated catheter body and extending from the proximal end to the expandable region, the control wire lumen adapted to receive a control wire (13) for effecting axial movement of the expandable region; and
a blind lumen (50) disposed in the elongated catheter body and extending from the proximal end and terminating at a position near the distal end of the expandable region, the blind lumen adapted to receive a radiation source wire (51), whereby the control wire is moved proximally to expand the expandable region into contact with the body lumen and permit perfusion of blood through the expandable region while the radiation source wire is inserted in the blind lumen to provide a radiation source to the body lumen.

2. An intravascular catheter for maintaining the patency of a body lumen for a period of time sufficient to permit delivery of a radiation dose to the body lumen, comprising:
an elongated catheter body (60) having a proximal end and a distal end and having an outer shaft (61) and an inner shaft (62) in a coaxial configuration;
an expandable region (66) disposed at the distal end of the elongated catheter body, the expandable region having a proximal end (65) and a distal end (69), the proximal end (65) of the expandable region being attached to a distal end (64) of the outer shaft, and the distal end (69) of the expandable region being attached to the distal end (68) of the inner shaft;
means for providing relative axial movement between the outer shaft and the inner shaft for expanding and contracting the expandable region;
a guide wire lumen (20) extending through the elongated catheter body from the proximal end to the distal end for receiving a guide wire (24); and
a blind lumen (50) disposed in the elongated catheter body and extending from the proximal end of the catheter body and terminating at a position near the distal end of the expandable region, the blind lumen adapted to receive a radiation source wire (51), whereby the outer shaft (61) is moved axially in relation to the inner shaft (62) to expand the expandable region into contact with the body lumen and permit perfusion of blood through the expandable region while the radiation source wire (51) is inserted in the blind lumen to provide a radiation source to the body lumen.

3. An intravascular catheter for maintaining the patency of a body lumen for a period of time sufficient to permit delivery of a radiation source to the body lumen, comprising:
an elongated catheter body having a proximal end and a distal end;
an expandable region disposed at the distal end of the elongated catheter body, the expandable region having a proximal end and a distal end;
a guide wire lumen extending through the elongated catheter body from the proximal end to the distal end for receiving a guide wire;
means for expanding and contracting the expandable region; and
a blind lumen disposed in the elongated catheter body and extending from the proximal end and terminating at a position near the distal end of the expandable region, the blind lumen adapted to receive a radiation source wire, whereby the means for expanding the expandable region is engaged to expand the expandable region into contact with the body lumen and permit perfusion of blood through the expandable region while the radiation source wire is inserted in the blind lumen to provide a radiation source to the body lumen.

4. The catheter of any of claims 1 to 3, wherein the expandable region (12) is formed from the distal end of the elongated catheter body (11) in a one-piece configuration.

5. The catheter of claim 4 when dependent on claim 1, wherein the control wire (13) is attached to the distal end (15) of the expandable region and withdrawing the control wire proximally will shorten the expandable region (12) thereby expanding it into contact with the body lumen, the distal end (15) of the expandable region moving axially in the proximal direction while the proximal end (14) of the expandable region remains fixed relative to the elongated catheter body.

6. The catheter of claim 2, wherein the relative axial movement between the outer shaft and inner shaft shortens or lengthens the expandable region thereby expanding it into contact with the inner wall of the body lumen or contracting it, respectively.

7. The catheter of claim 2, wherein the relative axial movement between the outer shaft and inner shaft is controlled by a rack and pinion.

8. The catheter of claim 2, wherein the relative axial movement between the outer shaft and inner shaft is controlled by ratcheting means.

9. The catheter of claim 2, wherein the relative axial movement between the outer shaft and inner shaft is controlled by gearing means.

10. The catheter of any of claims 1 to 3, wherein the radiation source wire has a proximal end and a distal end, the distal end having a radiation source (52) associated therewith.

11. The catheter of any of claims 1 to 3 wherein radiation source wire (51) delivers a low dosage of radiation to the body lumen at position adjacent the distal end of the blind lumen (50).

12. The catheter of claim 11, wherein the level of radiation delivered by the radiation source wire (51) is in the range of about 20 to 3000 rads over a period of no less than two minutes.

13. The catheter of any of claims 1 to 3, wherein the expandable region (12) is configured to center the radiation source wire within the body lumen so that substantially equal amounts of radiation energy are directed to the body lumen.

14. The catheter of claim 13, wherein the expandable region has a plurality of expandable members (12) disposed along the distal end of the elongated catheter body (11) to further center the radiation source wire (51) in the body lumen.

15. The catheter of any of claims 1 to 3, wherein the blind lumen (50) terminates in the distal end of the elongated catheter body (11) and does not open to the body lumen.

16. The catheter of any of claims 1 to 3, wherein the elongated catheter body is formed of a relatively stiff proximal section and a substantially flexible distal end.

17. The catheter of any of claims 1 to 3, wherein the proximal end of the elongated catheter body has a guide wire port (23) for receiving a guide wire (24) for over-the-wire delivery of the catheter.

18. The catheter of any of claims 1 to 3, wherein a guide wire port (21) for receiving a guide wire is disposed in a sidewall (22) of the catheter so that the catheter can be rapidly exchanged when it is withdrawn from the body lumen.

19. The catheter of claim 18, wherein a slit (25) is formed in the sidewall (22) of the elongated catheter body from the guide wire port (21) toward the distal end of the elongated catheter body (11) to facilitate rapid exchange upon withdrawing the catheter from the body lumen.

20. An intravascular catheter (10) for maintaining the patency of a body lumen for a period of time sufficient to permit delivery of a radiation source to the body lumen, comprising:
an elongated catheter body (11) having a proximal end and a distal end;
an expandable region (12) disposed at the distal end of the elongated catheter body, the expandable region having a proximal end (14) and a distal end (15);
a guide wire lumen (20) extending through the elongated catheter body from the proximal end to the distal end for receiving a guide wire (24);
a control wire lumen (17) disposed in the elongated catheter body and extending from the proximal end to the expandable region, the control wire lumen adapted to receive a control wire (13) for effecting axial movement of the expandable region; and
the expandable region having the radiation source (52) associated therewith so that the control wire can be moved axially to expand the expandable region into contact with the body lumen and provide perfusion of blood through the expandable region while the radiation source is being exposed to the body lumen.

21. The catheter of any of claims 1 to 3, wherein the radiation source wire (51) includes a radiation source (52) taken from the group of radiation sources having a half life of less than one hundred days.

22. The catheter of claim 20 or 21, wherein the radiation source (52) is taken from the group including iridium¹⁹², cobalt⁶⁰, vanadium⁴⁸, gold¹⁹⁸, and phosphorus³².

23. The catheter of claim 21 or claim 22, wherein the group of radiation sources (52) includes alpha-, beta- and gamma-emitting radiation.

24. The catheter of any of claims 1, 2, 3 or 20 wherein the body lumen is a coronary artery and the catheter is sized for intraluminal delivery into the coronary artery.

25. The catheter of claim 20, wherein at least a portion of the expandable region is formed from the radiation source.

26. The catheter of claim 20, wherein at least a portion of the expandable region is impregnated with the radiation source.

27. The catheter of claim 20, wherein at least a portion of the expandable region is coated with the radiation source.

28. The catheter of claim 20, wherein at least a portion of the expandable region is formed from a metal alloy taken from the group of metal alloys including stainless steel, tungsten, gold, palladium, platinum, tantalum, iridium, and nickel-titanium.

29. The catheter of claim 20, wherein at least a portion of the expandable region is formed from a polymeric material.

30. The catheter of claim 29, wherein the polymeric material is taken from the group of polymeric materials including polyethylene, polyethylene teraphthalate, and nylon.

31. A method for maintaining the patency of a body lumen for a period of time sufficient to permit delivery of a radiation source to the body lumen, comprising:
a) providing a catheter having:
an elongated catheter body (11) having a proximal end and a distal end;
an expandable region (12) disposed at the distal end of the elongated catheter body, the expandable region having a proximal end (14) and a distal end (15);
a guide wire lumen (20) extending through the elongated catheter body from the proximal end to the distal end for receiving a guide wire (24);
means for expanding and contracting the expandable region; and
a blind lumen (50) disposed in the elongated catheter body and extending from the proximal end and terminating at a position near the distal end (15) of the expandable region, the blind lumen adapted to receive a radiation source wire (51);
b) positioning the guide wire in the body lumen;
c) advancing the catheter over the guide wire by inserting the guide wire in the distal end of the catheter and into the guide wire lumen;
d) advancing the elongated catheter body over the guide wire until the expandable region is positioned in the body lumen;
e) expanding the expandable region into contact with the body lumen;
f) centering the blind lumen in the body lumen;
g) perfusing blood flow through the expandable region;
h) inserting the radiation source wire in the blind lumen for delivering a radiation dose to the body lumen;
i) contracting the expandable region; and
j) withdrawing the catheter and the radiation source wire from the body lumen.
